Europäisches Patentamt

European Patent Office

Office européen des brevets

(11) Publication number: **0 111 842**
**A2**

# EUROPEAN PATENT APPLICATION

(21) Application number: **83112371.6**

(22) Date of filing: **08.12.83**

(51) Int. Cl.³: **A 61 M 5/14**

(30) Priority: **16.12.82 US 450172**

(43) Date of publication of application:
**27.06.84 Bulletin 84/26**

(84) Designated Contracting States:
**DE GB**

(71) Applicant: CHESEBROUGH-POND'S INC.
33 Benedict Place
Greenwich, Conn. 06830(US)

(72) Inventor: Srebnik, Jack
221 Highwoods Drive
Guilford, CT 06437(US)

(72) Inventor: Timothy, Earl J.
23 Riverside Drive Apt. A11
Clinton, CT 06437(US)

(72) Inventor: Dana, Duncan L.
10-2 Browns Lane
Old Lyme, CT 06371(US)

(74) Representative: Patentanwälte Grünecker, Dr.
Kinkeldey, Dr. Stockmair, Dr. Schumann, Jakob, Dr.
Bezold, Meister, Hilgers, Dr. Meyer-Plath
Maximilianstrasse 58
D-8000 München 22(DE)

(54) **Gravity flow controller.**

(57) A clamping arrangement for a flow controller comprises a barrel shaped cam which is eccentrically rotatable to cooperate with a block to control flow through a flexible tubing positioned between the cam and the block. The shape of the block corresponds to that of the cam so that they fit flush together when in contact. Rotation about the eccentric axis of the cam is achieved by a motor on which the cam is mounted, which is itself controlled by a microprocessor in the flow controller in response to selected inputs thereto to achieve a predetermined flowrate through an IV administration set type flexible tubing.

./...

EP 0 111 842 A2

FIG. 1

172

13   11

9

15

3
85
1
5   75

7

19

# GRAVITY FLOW CONTROLLER

## BACKGROUND OF THE INVENTION

This invention relates to a flow controller which automatically regulates the flow through a gravity-fed infusion line of the type for use in intravenous (IV) administrations. More particularly, the invention relates to an infusion controller having an improved clamping mechanism for regulating the flow of a solution through a flexible tubing.

Generally, the term "infusion pump" is often used to describe all powered infusion control devices. However, as employed in this application, this term is not intended to include devices employing a pump to achieve infusions. Instead, this term will generally be used to describe those devices which employ a clamping mechanism in a gravity-fed system. The distinctions between the two types of devices are discussed in Health Devices, March-April 1979, Vol. 8, Nos. 5-6, pages 103-130, and January-February 1982, pages 75-95, whose disclosures are specifically incorporated by reference herein.

The prior art IV controllers have generally been of the drop-controller type which control the rate at which drops of fluid flow to the patient. Such drop controllers rely on clamping of a conduit to restrict the rate at which fluid flows therethrough. One such device is presently manufactured and sold by IVAC corporation and involves a pincher-anvil clamping mechanism wherein the administration set tubing is installed between one wedge-like projection and one flat plate that intermittently compresses and occludes the tubing.

However, the prior art clamping type devices suffer the disadvantage that the clamping has been frequently unreliable in controlling the rate of fluid flow. More specifically, the prior art clamping devices suffer from problems in wearing of the tubing during a normal period of use, often resulting in compromised performance. Furthermore, the precise adjustment of the clamping mechanism to achieve a desired flowrate has been difficult.

One attempt in the prior art to remedy these problems is discussed in U.S. 4,314,567 and involves employing a disposable cassette through which the fluid flows to the patient. More specifically, a disposable cassette which passes fluid at a controlled rate is associated with an IV controller and has the fluid feed tubing connected thereto. However, this type of device has the disadvantage that it is significantly more complicated than the simple clamping arrangements. Moreover, this type of device requires a specific type IV administration kit, i.e., tubing, and cannot be used with most existing IV kits. Furthermore, both the clamp type as well as the various other types of devices, i.e. cassette, require either a very complicated arrangement for metering out individual drops or, in the case of the clamping type device, complete opening and closing of the tube is required to create a drop which, in these type devices often consumes considerable amounts of power to achieve the solution metering desired.

Other prior art devices are disclosed in U.S. 4,336,800, U.S. 4,299,218, U.S. 4,207,871, U.S. 4,204,538, U.S. 4,137,913 and U.S. 4,037,598. However, all of these devices suffer from one or more disadvantages of the prior art as discussed above. 0111842

It is thus an object of the invention to provide an IV controller having an improved clamping mechanism for use with tubing on most IV administration kits.

It is another object of the invention to provide an improved clamping mechanism for use in IV controllers which can be used with numerous types of existing tubing to provide highly precise metering of a solution to a patient.

Still another object of the present invention is to provide an IV controller having an improved clamping mechanism which provides more accurate metering in a very simple manner.

Upon further study of the specification and appended claims, further objects and advantages of this invention will become apparent to those skilled in the art.

## SUMMARY OF THE INVENTION

In one aspect the invention comprises an improvement to a flow controller of the type having a clamping mechanism for IV administration set type flexible tubing. More specifically, the controller includes a clamping arrangement for regulating the opening and closing of the tubing to ensure a desired flow therethrough. This control arrangement is connected to a master control and a selecting unit through which the controller operator selects the desired flow rate. The improved clamping mechanism includes an eccentrically rotatable generally barrel shaped cam with the axis of rotation thereof being slightly off-center from the central axis of the cam. A motor having the cam connected thereto is associated with the control arrangement, and is adapted for rotating the cam to a selected one of a plurality of different positions between a fully open and a fully closed position. A block is positioned with respect to the cam for having tubing passed therebetween to enable the discussed clamping between open, partially open and closed conditions of the tubing with the cam. The block has a surface shaped correspondingly opposite to the shape of the cam to ensure self-centering of the tubing thereon.  Furthermore, the shape of the block is such that it fits flush against the cam when in contact.

In another aspect, the invention comprises the clamp adapted for use with a flow controller as discussed above which includes the described cam which is adjustable throughout different settings for precisely metering the flow of liquid through an IV administration type flexible tubing.

## BRIEF DESCRIPTION OF THE DRAWINGS

Various other objects, features and attendant advantages of the present invention will be more fully appreciated as the same becomes better understood when considered in conjunction with the accompanying drawings, in which like reference characters designate the same or similar parts throughout the several views, and wherein:

Figure 1 is a cross-sectional side view of an IV controller showing the improved clamping mechanism mounted therein;

Figure 2 is a cross-sectional front view of the device of Figure 1 showing the entire clamping mechanism mounted therein;

Figure 3 is an enlarged cross-sectional partial side view of the clamping mechanism mounted in the IV controller;

Figure 4A and 4B are side and front views respectively of the barrel shaped cam of the clamping device;

Figure 5 is a front view of the block of the clamping device;

Figure 6 is a general front view of the IV controller showing the control panel;

0111842

Figure 7 is a cross-sectional top view of the IV controller showing the clamp for mounting on the IV pole; and

Figure 8 is a block diagram generally showing the arrangement of the various systems of the IV controller to more clearly understand the operation thereof.

## DETAILED DISCUSSION OF THE INVENTION

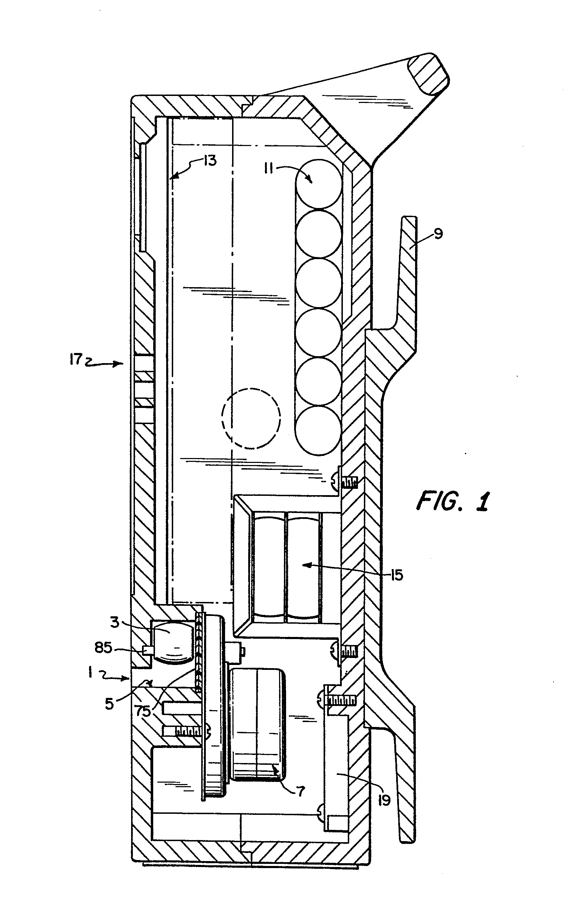

The improved IV controller according to the invention is shown in Figure 1 in a cross-sectional view with the clamping arrangement 1 shown mounted therein. This clamping arrangement 1 includes a cam 3 spaced from a block 5 (not shown in this view) such that the tubing from an IV administration kit passes therethrough between the cam 3 and block 5.

The cam 3 is mounted for rotation on a motor 7 by means of a "D" shaped rod 85 extending into the cam 3 and secured to the cam 3 by a screw or pin 85'. Typically, this motor 7 will be a stepping motor, as conventionally known to those skilled in the art, capable of moving the cam 3 between the two positions, i.e., a fully open and fully closed position, in step-like increments in response to control signals transmitted thereto. The motor 7 moves the cam 3 to predetermined fixed fully open, partially open, or closed positions to ensure achievement of selected flowrates through an IV tubing therein. Between the motor 7 and the cam 3 is a gasket 75, typically a silicone gasket, which seals the tubing passageway from the internal portions of the IV controller.

As generally schematically shown in Figure 1, the IV controller will generally be powered by conventional house current through a power cord (not shown), the excess length of which is normally wound around a bracket 9 attached to the IV controller. In the event power goes out, in order to ensure that the IV controller continues regulating the IV flow through the tubing, a back-up power supply 11, typically comprised of a battery of 6 nickel-cadmium cells, is provided housed in the controller itself. The internal back-up power supply 11 is conventional and as in the prior art is typically capable of maintaining the IV controller in operation for at least four hours of normal operation.

To monitor and control the operation of the IV controller is a printed circuit 13 making up part of a preprogrammed microprocessing arrangement, and is associated with a transformer 15 which supplies low voltage alternating current to the printed circuit 13 for circuit power.

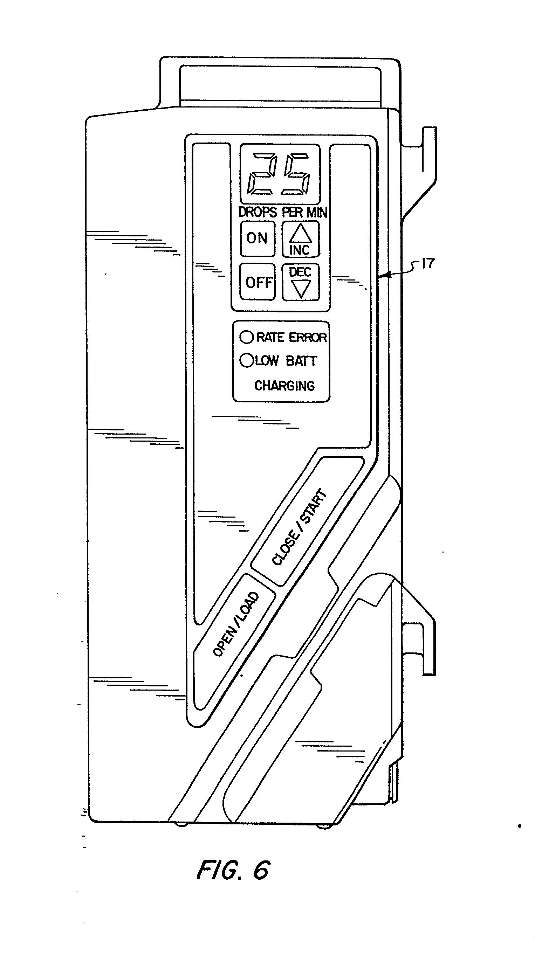

As shown in the front view of Figure 6, various operator control buttons on a control panel 17 are provided which are associated with the printed circuit 13 for the operator to select the desired flowrate to cause the printed circuit 13 provide the necessary signals to cause the motor 7 to move the cam 3 into a position corresponding, in a preprogrammed manner, to the selected drop or flowrate using input from the drop sensor. All of these systems are conventional and are more specifically discussed in the above incorporated by reference Health Devices articles, and the cited patents.

Various safety and monitoring sensors are provided, as well known from the above-discussed publications whose teachings are incorporated by reference herein, and are not shown for the sake of clarity. For instance, IV controllers should remain accurate to about $\pm$ 10%. To ensure accurate flowrates, a drop or flowrate sensor is provided associated with the control circuitry and IV tubing. Likewise, the controller system will include a safeguard system to prevent uncontrolled flow to the patient during set up, operation, and IV set

removal. Implementation of all of these features is well known to those skilled in the art and will not be discussed in detail herein since they are also discussed in the above cited article "Infusion Controllers", Health Devices, January-February 1982 at pages 81-83. In the event improper conditions or tampering occurs, the IV controller includes an audio alarm 19 which is activated in a preprogrammed manner by the printed circuit 13 upon the occurrence of these conditions.

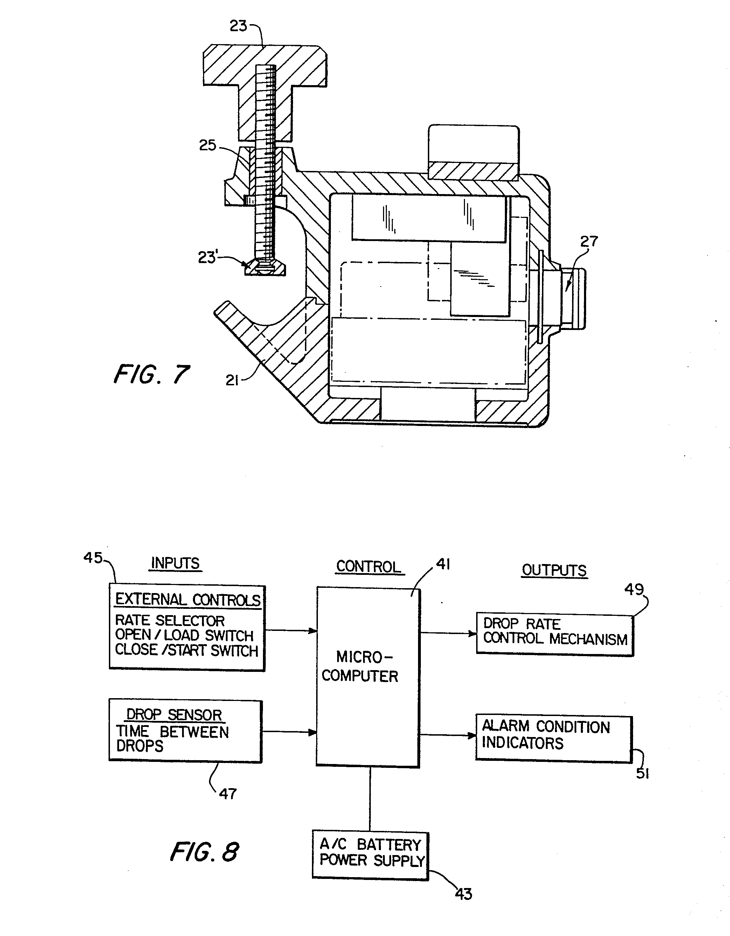

The IV controller further shows in Figure 7 a conventional mounting arrangement 21 wherein a clamping screw 23 passes through a bracket 25 so that the end 23' of the screw 23 cooperates with bracket 25 to mount the controller to an IV pole in association with an IV administration set. Also shown is the connecting positioning of a flow sensor 27 on the controller. This sensor is also conventional in nature as previously discussed.

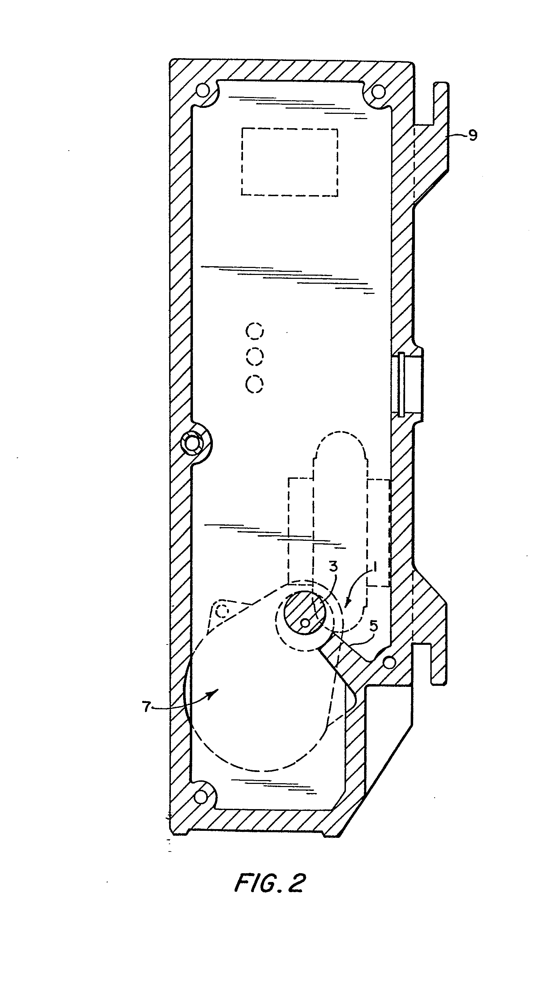

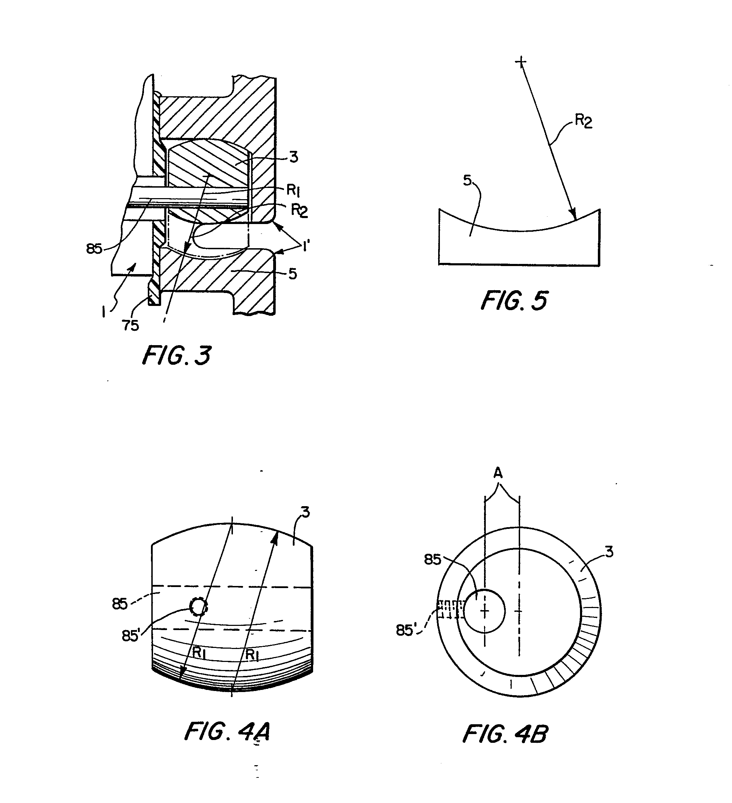

The clamping arrangement 1 for the IV administration tubing is more clearly shown in Figures 2 and 3. More specifically, the clamp 3 is shown to be generally barrel shaped and mounted off-center to the shaft of the motor 7 so that the cam 3 is eccentrically rotated in a step-like manner by the motor 7 from a maximum spacing from the block 5 to a fully closed clamping position in contact with the block 5. Various intermediate partially blocking positions are possible with a stepping motor 7, and the desired position will be achieved in accordance with a selected flowrate in a preprogrammed manner as discussed above.

As shown in Figures 2, 3 and 5, the block 5 employed is generally plano-concave and has a surface spaced opposite to the clamping surface of the cam 3 which is generally shaped correspondingly opposite to the clamping surface of the cam 3 so that they fit flush together when the cam 3 is rotated to the fully closed position. Furthermore, as a result of its shape the block 5 ensures self-centering of the IV tubing when associated therewith, thereby precluding improper closing and opening control of the tubing as a result of the tubing being partially out of the clamping surface.

With reference to the specific dimensions of the block 5 and cam 3, for use with most conventional IV set, the exact configurations are more clearly shown in Figures 4A, 4B and 5. It is preferred that the barrel shaped cam have a diameter of .500 in. at its maximum circumference, and a width from flat surface to flat surface of .500 in. The radius of curvature $R_1$ of the curved surfaces should also be .500 in. and the center of shaft 85 is mounted a distance A of about .100 in. off-center of the cam 3. The block 5 has similar dimensions but the radius of curvature $R_2$ of the contact surface is reversed to that of the cam 3, i.e., it is shaped inwardly toward the center of the block 5. Furthermore, when mounted in the controller the block 5 is fixed in position as shown in Figure 2.

The mounting of the cam 3 with respect to the block 5 is such as to provide a spacing 1', as shown in Figure 3, to accommodate tubings of up to 0.25 inch in diameter when in fully open position, and at the same time to have the cam 3 contact the block 5 when in fully closed position. It is preferred that the cam 3 be made of stainless steel while the block 5 is resilient plastic molded as part of the controller case. Alternatively, the block 5 and cam 3 can be of aluminum with the block 5 mounted by a pin on the controller. Other equivalent materials as well known to those skilled in the art can be employed.

Figure 8 is a block diagram illustrating generally the operation of the IV controller. More particularly, the entire system is controlled generally by a microcomputer 41 which is preprogrammed and is conventional

0111842

as discussed with reference to the prior art. This microcomputer is powered by a power supply 43 which is an external AC power supply or internal batteries as previously discussed, and is adapted for processing externally applied signals from a control panel 45 from the operator, and from a drop sensor 47 to control the drop rate control mechanism 49, i.e., clamping arrangement and the alarm condition indicator 51 under predetermined operating conditions.

From the foregoing description, one skilled in the art can easily ascertain the essential characteristics of this invention, and without departing from the spirit and scope thereof, can make various changes and modifications of the invention to adapt it to various usages and conditions.

WHAT IS CLAIMED IS:

1. In a motor driven flow controller adapted for automatically regulating a gravity induced liquid flow through flexible tubing of the IV infusion type and comprising clamping means to constrict the tubing for controlling the flow of liquid through the tubing, control means associated with the clamping means for activating the clamping means, and selecting means associated with the control means for selecting a desired flowrate of liquid through the tubing and for causing the control means to control the clamping means to achieve the selected flowrate through the tubing, the improvement wherein the clamping means comprises an eccentrically rotatable generally barrel shaped cam with the axis of rotation being slightly off-center from the central axis of said cam; motor means having said cam connected thereto associated with said control means and adapted for rotating said cam to a selected one of a desired plurality of different positions about its axis of rotation; and a block positioned with respect to said cam for having a tubing passed therebetween for clamping said tubing between open, partially open and closed positions, and said block having a surface correspondingly shaped opposite to the shape of said cam to ensure self-centering of the tubing thereon with said surface being such that said cam fits flush against said block when in the fully closed position.

2. A flow controller according to claim 1, wherein said motor means comprises a stepping motor adapted for rotating said cam in predetermined fixed incremental amounts.

3. A flow controller according to claim 1, wherein said control means comprises a preprogrammed microprocessor adapted for causing said motor means to rotate said cam to control positions in response to externally applied signals corresponding to selected flowrates through a tubing associated therewith.

4. A flow controller according to claim 3 further comprising a flowrate sensor associated with said preprogrammed microprocessor for providing information corresponding to the rate of flow through a tubing to the microprocessor, and whereby said microprocessor is adapted to process said information and to cause said control means to adjust the position of said cam in response to said information to ensure a correct flow of fluid through a flexible tubing associated therewith.

5. A flow controller according to claim 1, wherein the spacing between said cam and said block in the fully open position is such as to permit positioning of an IV type flexible tube of up to 0.25 inches in diameter between said cam and block.

6. A flow controller according to claim 1, wherein said cam is formed of stainless steel and said block is formed of resilient plastic.

7. A flow controller according to claim 6, wherein said block is molded integral to the controller housing.

0111842

8. A flow controller according to claim 4 further comprising alarm means to cause the controller to shutoff flow through the tubing and to transmit an alarm signal under abnormal flow conditions.

9. A flow controller according to claim 7, wherein said controller is adapted for use with an IV infusion kit.

10. A clamping mechanism adapted for use with a flow controller for regulating gravity induced liquid flow through a flexible tubing of the IV infusion type by constricting the tubing to control the flow of liquid through the tubing, the mechanism comprising: an eccentrically rotatable generally barrel shaped cam with the axis of rotation being slightly off-center from the central axis of said cam; rotating means having said cam connected thereto and adapted for rotating said cam to selected fixed positions about its axis of rotation; and a block positioned with respect to said cam for having a tubing passed therebetween for clamping said tubing between open, partially open and closed positions, and said block having a surface correspondingly shaped opposite to the shape of said cam to ensure self-centering of the tubing thereon with said surface being such that said cam fits flush against said block when in the fully closed position.

1/5

0111842

FIG. 1

FIG.2

0111842

FIG. 3

FIG. 5

FIG. 4A

FIG. 4B

FIG. 6

5/5

0111842

FIG. 7

FIG. 8